# FASCICULE DE BREVET EUROPEEN

(11) **EP 0 539 450 B1**
(45) Date de publication et mention de la délivrance du brevet: **30.03.1994**
(21) Numéro de dépôt: 91913246.4
(22) Date de dépôt: 15.07.1991
(51) Int. Cl.: C07D 498/18

(54) **PROCEDE DE PREPARATION DE SULFINYL PRISTINAMYCINE II B**
VERFAHREN ZUR HERSTELLUNG VON SULFINYL-PRISTINAMYCIN IIB
METHOD FOR THE PREPARATION OF SULPHINYL PRISTINAMYCIN II B

(30) Priorité: 16.07.1990 FR 9009034
(43) Date de publication de la demande: 05.05.1993
(73) Titulaire: RHONE-POULENC RORER S.A., 92160 Antony (FR)
(72) Inventeur: RADISSON, Xavier, F-69006 Lyon (FR)
(74) Mandataire: Savina, Jacques
(86) Numéro de dépôt international: FR9100580
(87) Numéro de publication internationale: WO9201693

(56) Documents cités:
- EP-A- 0 191 662
- EP-A- 0 252 720
- GB-A- 2 206 577
- GB-A- 2 206 879
- US-A- 4 775 753

## Description

Les (dialcoylamino-2 alcoyl) sulfinyl-26 pristinamycine II_{B} de formule générale : 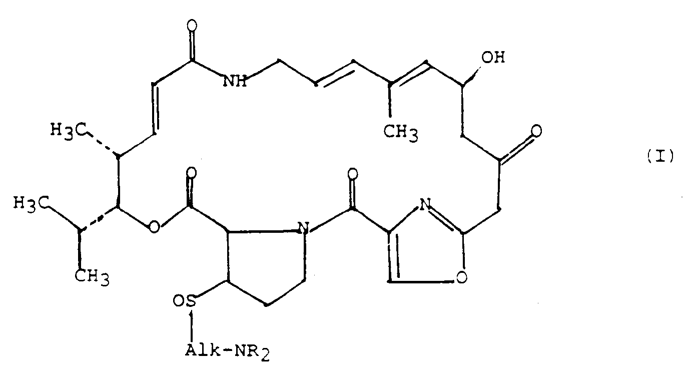
dans laquelle Alk représente un radical aicoylène droit ou ramifié et R représente des radicaux alcoyle droits ou ramifiés, ces radicaux contenant 1 à 10 atomes de carbone, sont des produits connus pour leur activité antibactérienne et leur action synergisante de l'activité antibactérienne de la pristinamycine I_{A} ou encore comme intermédiaires de synthèse pour préparer les sulfones correspondantes, comme cela a été décrit dans le brevet européen 191 662.

Les dérivés de la pristinamycine II_{B} de formule générale (I) peuvent être obtenus par oxydation du sulfure correspondant notamment selon l'enseignement du brevet européen 191 662 qui décrit l'oxydation d'un sulfure de formule : 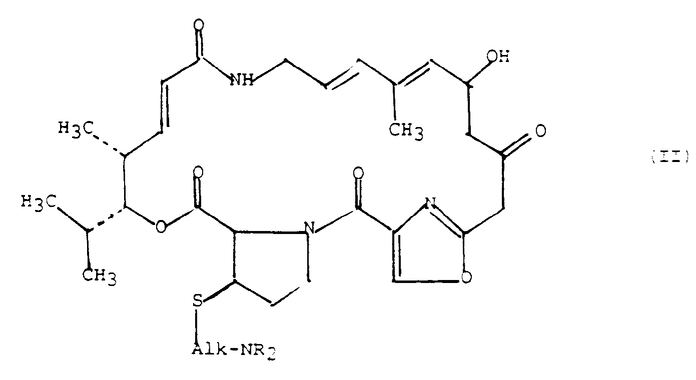
dans laquelle Alk et R sont définis comme précédemment, par un péracide organique ou minéral.

Le brevet américain US 4 775 753 décrit également l'obtention de (dialcoylamino-2 alcoyl) sulfinyl-26 pristinamycine II_{B} de formule générale (I) à partir de (dialcoylamino-2 alcoyl) thio-26 pristinamycine II_{B} de formule générale (II) par oxydation par l'oxone.

La demande de brevet GB 2 206 577 décrit l'oxydation de sulfures de formule générale (II) en sulfones.

Il a maintenant été trouvé que la (dialcoylamino-2 alcoyl) sulfinyl-26 pristinamycine II_{B} de formule générale (I) peut être obtenue par oxydation du sulfure correspondant de formule générale (II), par l'eau oxygénée, en présence de tungstate de sodium, en opérant en milieu alcoolique à une température comprise entre -25 et 25°C.

Il est nécesaire que l'oxydant soit introduit en excès par rapport à la quantité de produit à oxyder. L'eau oxygénée employée peut être introduite à raison de 2 à 5 équivalents. Le plus généralement elle est employée à raison d'environ 2 équivalents par mole de sulfure de pristinamycine II_{B}.

Il est entendu que la température de réaction sera fixée en fonction du nombre d'équivalents d'oxydant investis. Lorsque l'on opère en présence de 2 équivalents d'eau oxygénée, la température peut varier de -25 à +25°C. Lorsque l'on opère en présence de 5 équivalents d'oxydant, la réaction est mise en oeuvre entre -25 et -10°C.

Le solvant alcoolique préféré est l'éthanol, mais il est également possible d'opérer dans le méthanol ou l'isopropanol.

Ce procédé présente l'avantage de donner accès à un produit de meilleur degré de pureté en évitant les purifications par chromatographie, et de ce fait permet une amélioration très nette du rendement. De plus, lorsque le produit de formule (I) ainsi obtenu, doit être utilisé ultérieurement pour la préparation de (dialcoylamino-2 alcoyl) sulfonyl-26 pristinamycine II_{B} de formule générale : 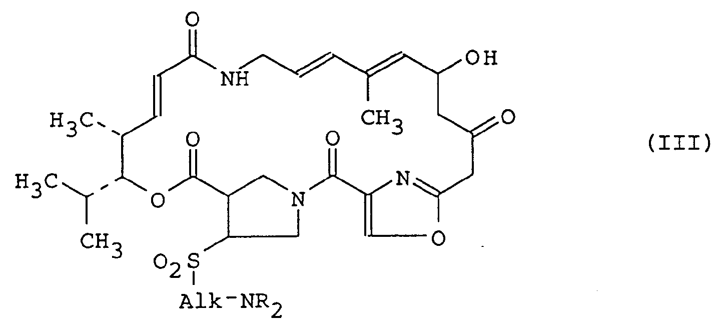
dans laquelle Alk et R sont définis comme précédemment, le dérivé de pristinamycine II_{B} de formule générale (I) est de qualité suffisante pour pouvoir utiliser directement le produit brut dans l'étape ultérieure d'oxydation, par exemple selon le procédé de la demande de brevet européen 252 720, sans qu'il soit nécessaire d'effectuer préalablement une chromatographie, une recristallisation ou une filtration sur silice comme c'était le cas pour les procédés antérieurement connus. Il en résulte un rendement global amélioré et également une simplification de la mise en oeuvre du procédé.

Les exemples suivants, illustrent la présente invention.

### EXEMPLE 1

2,635 g (4 mM) de (diéthylamino-2 éthyl) thio-26 pristinamycine II_{B} isomère A en suspension dans 16 cm3 d'éthanol absolu, sont placés dans un bain de glace. On ajoute alors, en 2 minutes, à une température de 1 à 2°C, une solution de 13,2 mg (0,04 mM ; 1 % molaire) de tungstate de sodium dihydraté dans 0,906 g (8 mM, 2 équivalents) d'eau oxygénée (30 %). La suspension de sulfure devient limpide. L'agitation est poursuivie pendant 1 heure et 5 minutes au total.

A 0°C, 5 cm3 d'eau sont additionnés au mélange réactionnel. Après 5 minutes d'agitation, la solution est extraite par 40 cm3 de chlorure de méthylène. La phase organique est lavée 4 fois par 5 cm3 d'eau et séchée sur sulfate de sodium. Après concentration à sec, 2,585 g d'un solide blanc crème sont obtenus, correspondant à la (diéthylamino-2 éthyl) sulfinyl-26 pristinamycine II_{B} et titrant 4 % en isomère A₁, 90,3 % en isomère A₂ (RR en sulfoxyde : 90,3 %).

### EXEMPLE 2

1,98 g (3 mM) de (diéthylamino-2 éthyl) thio-26 pristinamycine II_{B} isomère A en suspension dans 12 cm3 d'éthanol est refroidi à -20°C et une solution de 9,9 mg (0,03 mM, 1 % molaire) de tungstate de sodium dihydraté dans 1,7 g (15 mM, 5 éq.) d'eau oxygénée à 30 % y est additionnée en 3 minutes, sous agitation.

La température du mélange réactionnel est maintenue entre -15 et -20°C pendant 1 heure 30 minutes d'agitation et 3 cm³ d'eau sont ajoutés en laissant la température remonter à -10°C. 25 cm³ de dichlorométhane sont ajoutés, la phase organique est décantée à 0°C, lavée par 5 fois 5 cm³ d'eau, séchée sur sulfate de sodium et concentrée à sec sous pression réduite pour founir la (diéthylamino-2 éthyl) sulfinyl-26 pristinamycine II_{B} mélange des isomères A₁ et A₂ (A₁ 4 % ; A₂ 96 %) attendue avec un rendement de 88 %.

## Revendications

1. Procédé de préparation de (dialcoylamino-2 alcoyl) sulfinyl-26 pristinamycine II_{B} de formule générale : 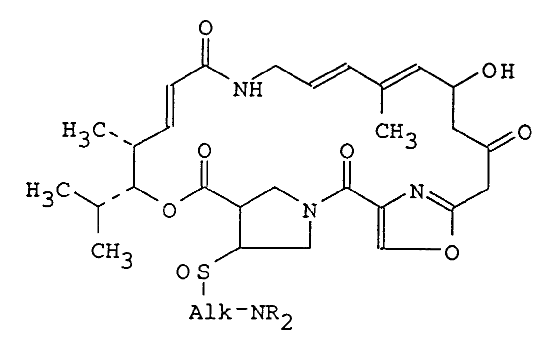 dans laquelle Alk représente un radical alcoylène droit ou ramifié et R représente des radicaux alcoyle droits ou ramifiés, ces radicaux contenant 1 à 10 atomes de carbone, par oxydation de la (dialcoylamino-2 alcoyl) thio-26 pristinamycine II_{B} correspondante, de formule générale : 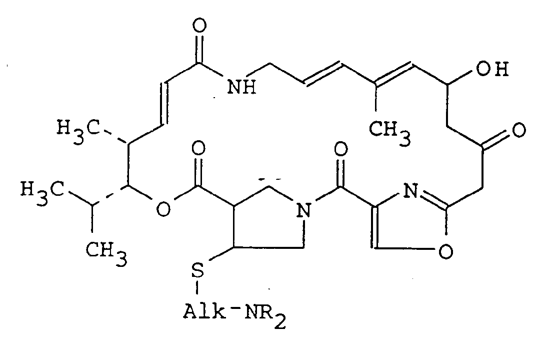 dans laquelle Alk et R sont définis comme ci-dessus, par 2 à 5 équivalents d'eau oxygénée en présence de tungstate de sodium, en milieu alcoolique, à une température variant entre -25 à -10°C pour 5 équivalents d'eau oxygénée, et -25 à +25°C pour 2 équivalents d'eau oxygénée.

## Patentansprüche

1. Verfahren zur Herstellung von 26-(2-Dialkylaminoalkyl)-sulfinyl-pristinamycin II_{B} der allgemeinen Formel 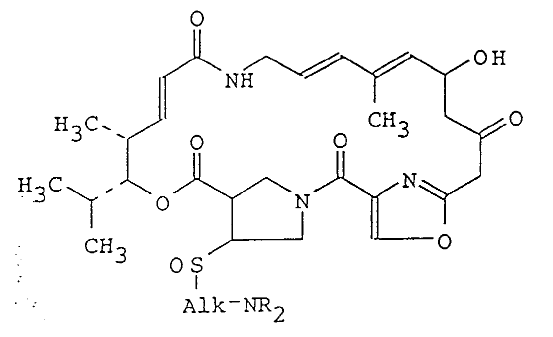 worin Alk für einen geradkettigen oder verzweigten Alkylenrest steht und R geradkettige oder verzweigte Alkylreste bedeutet, wobei diese Reste 1 bis 10 Kohlenstoffatome enthalten, durch Oxidation des entsprechenden 26-(2-Dialkylaminoalkyl) -thio-pristinamycin II_{B} der allgemeinen Formel 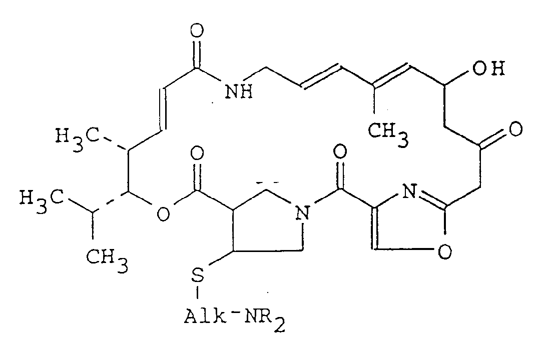 worin Alk und R wie vorstehend definiert sind, mit 2 bis 5 Äquivalenten Wasserstoffperoxid in Gegenwart von Natriumwolframat in alkoholischem Milieu bei einer Temperatur, variierend zwischen -25 und -10°C für 5 Äquivalente Wasserstoffperoxid und -25 bis +25°C für 2 Äquivalente Wasserstoffperoxid.

## Claims

1. Process for the preparation of 26-((2-dialkylaminoalkyl)sulphinyl)pristinamycin II_{B} of general formula: 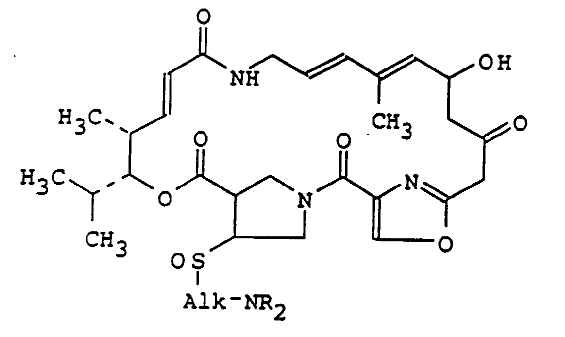 in which Alk represents a straight or branched alkylene radical and R represents straight or branched alkyl radicals, these radicals containing 1 to 10 carbon atoms, by oxidation of the corresponding 26-((2-dialkylaminoalkyl)thio)pristinamycin II_{B} of general formula: 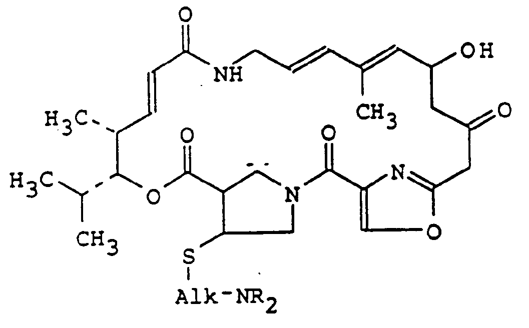 in which Alk and R are defined as above, with 2 to 5 equivalents of hydrogen peroxide in the presence of sodium tungstate, in alcoholic medium, at a temperature varying between -25 and -10°C for 5 equivalents of hydrogen peroxide and -25 and +25°C for 2 equivalents of hydrogen peroxide.
